# EUROPÄISCHE PATENTSCHRIFT

(11) **EP 4 188 675 B1**
(45) Veröffentlichungstag und Bekanntmachung des Hinweises auf die Patenterteilung: **12.06.2024**
(21) Anmeldenummer: 21748853.5
(22) Anmeldetag: 22.07.2021
(51) Int. Cl.: B29C 49/42, B29C 49/64, B29C 49/06, B29K 67/00, B29L 31/00

(54) **VORRICHTUNG ZUR BEHANDLUNG VON BEHÄLTERROHLINGEN**
DEVICE FOR TREATING CONTAINER BLANKS
DISPOSITIF DE TRAITEMENT D'ÉBAUCHES DE RÉCIPIENTS

(30) Priorität: 31.07.2020 DE 102020120284
(43) Veröffentlichungstag der Anmeldung: 07.06.2023
(73) Patentinhaber: KRONES AG, 93073 Neutraubling (DE)
(72) Erfinder: KAISER, Alexander, 93073 Neutraubling (DE)
(74) Vertreter: v. Bezold & Partner Patentanwälte - PartG mbB
(86) Internationale Anmeldenummer: PCT/EP2021/070540
(87) Internationale Veröffentlichungsnummer: WO 2022/023164

(56) Entgegenhaltungen:
- EP-A1- 2 848 382
- DE-A1-102010 018 153
- US-A1- 2003 230 941
- US-A1- 2019 099 936

## Beschreibung

### Technisches Gebiet

Die Erfindung betrifft eine Vorrichtung zur Behandlung von Behälterrohlingen für eine Behälterbehandlungsmaschine.

### Technischer Hintergrund

Dünnwandige Kunststoffbehälter aus thermoplastischem Kunststoffmaterial wie bspw. PET werden üblicherweise aus spritzgegossenen Vorformlingen / Behälterrohlingen, sog. Preforms, hergestellt und in einem zweistufigen Streckblasverfahren zu Behältern umgeformt. Bei der Verarbeitung werden die Behälterrohlinge auf eine definierte Prozesstemperatur erwärmt, um den Umformvorgang beim Streckblasen zu ermöglichen. Dabei werden die Behälterrohlinge zunächst in einer Heizstrecke beheizt, um die relativ dickwandigen Behälterrohlinge über ihren gesamten Querschnitt ausreichend zu erwärmen, so dass mit dem anschließenden Umformprozess mittels eines Streckblasverfahrens ein Hohlkörper geformt werden kann.

Aus dem Stand der Technik sind Vorrichtungen zum Blasformen von Gegenständen aus vorgefertigten Behälterrohlingen bekannt, wie etwa aus der DE 10 2010 018 153 A1.

Herkömmlich können die Behälterrohlinge an einer Kette gezogen durch den Ofen transportiert werden. Wenn jedoch stromaufwärts des Ofens Behälterrohlinge aufgrund von Beschädigung ausgeschleust werden müssen, kann dies zur Folge haben, dass beim Transport mit der Kette eine oder mehrere große Lücken zwischen den Behälterrohlingen durch den Ofen transportiert werden. Dies kann dazu führen, dass die der Lücke benachbarten Behälterrohlinge mehr Hitze abbekommen und der Blasprozess mit diesen Behälterrohlingen instabil werden würde. Somit ist man ggf. gezwungen, die Behälterrohlinge neben der Lücke nach dem Heizen vor dem Blasprozess auszusondern. Das kann zur Folge haben, dass die Lücke im Behälterrohlingstrom / Behälterstrom größer wird und wieder technisch aufwendig geschlossen werden muss.

Die EP 2 848 382 A1 offenbart eine Behälterbehandlungsanlage mit einem Ofen und einer dem Ofen nachgeschalteten Blasformvorrichtung zum Bearbeiten einer Vielzahl von Behälterrohlingen, wie Preforms, mit einer Transporteinrichtung, die zum Fördern mehrerer Träger ausgebildet ist.

Jeder Träger ist zur Aufnahme zumindest eines Behälterrohlings vorgesehen, wobei die Transporteinrichtung und der Träger so aufeinander abgestimmt sind, dass jeder Träger individuell durch den Ofen fahrbar ist.

Die US 2019/099936 A1 offenbart eine Vorrichtung zum Behandeln von Behältern, mit einer ersten Transporteinrichtung, die einen durchgehenden Träger aufweist, auf dem eine erste Mehrzahl von Transportelementen beweglich angeordnet ist, die jeweils mindestens eine Halterung zum Halten von Kunststoffvorformlingen aufweisen und zum Transport dieser Kunststoffvorformlinge entlang eines ersten Transportweges ausgebildet und konfiguriert sind, und mit mindestens einer entlang des ersten Transportweges angeordneten Heizeinrichtung, um die Kunststoffvorformlinge während ihres Transports entlang des ersten Transportweges zumindest abschnittsweise zu erwärmen.

Die US 2003/230941 A1 offenbart ein gesteuertes Bewegungssystem, das einen Läufer aufweist, der beweglich auf einer Schiene montiert ist, um sich entlang eines Weges zu bewegen. Aktive und reaktive Elemente sind mit der Schiene und dem Läufer verbunden und sind für den Antrieb und die Steuerung des Läufers entlang des Weges konfiguriert.

Der Erfindung liegt die Aufgabe zu Grunde, eine alternative und/oder verbesserte Vorrichtung zur Behandlung von Behälterrohlingen zu schaffen.

### Zusammenfassung der Erfindung

Die Aufgabe wird gelöst durch die Merkmale des unabhängigen Anspruchs 1. Vorteilhafte Weiterbildungen sind in den abhängigen Ansprüchen und der Beschreibung angegeben.

Ein Aspekt der vorliegenden Offenbarung betrifft eine Vorrichtung zur Behandlung von Behälterrohlingen für eine Behälterbehandlungsanlage (z. B. zum Herstellen, Reinigen, Prüfen, Abfüllen, Verschließen, Etikettieren, Bedrucken und/oder Verpacken von Behältern für flüssige Medien, vorzugsweise Getränke oder flüssige Nahrungsmittel). Die Vorrichtung weist einen Ofen zum Erwärmen der Behälterrohlinge auf. Die Vorrichtung weist ein Planarantriebssystem aufweisend ein Grundelement und mehrere Bewegungsvorrichtungen zum Transportieren der Behälterrohlinge auf. Die mehreren Bewegungsvorrichtungen sind bezüglich des Grundelements unabhängig voneinander bewegbar, vorzugsweise mittels magnetischer Wechselwirkung zwischen dem Grundelement und den mehreren Bewegungsvorrichtungen. Die Behälterrohlinge sind mittels der mehreren Bewegungsvorrichtungen durch den Ofen bewegbar.

Vorteilhaft ermöglicht die Vorrichtung eine kompaktere Konstruktion des Ofens. Das Planarantriebssystem kann quasi verschleißfrei betrieben werden. Das Planarantriebssystem ermöglicht zudem eine flexible Bewegung der Behälterrohlinge durch den Ofen. Lücken im Behälterrohlingstrom können beispielsweise flexibel geschlossen werden. Dadurch kann bevorzugt eine Ausschussrate gesenkt werden. Auch kann die Länge der Heizstrecke durch den Ofen aufgrund der Flexibilität der Bewegung der Bewegungsvorrichtungen verändert werden und so bspw. an einen Durchsatz oder eine Leistung der Vorrichtung angepasst werden.

Bevorzugt können die Bewegungsvorrichtungen außerhalb des Ofens bewegt werden, während die Bewegungsvorrichtungen die Behälterrohlinge durch den Ofen bewegen.

In einem Ausführungsbeispiel ist das Grundelement oberhalb des Ofens angeordnet, und die mehreren Bewegungsvorrichtungen sind über Kopf an einer Unterseite des Grundelements bewegbar. Alternativ kann bspw. das Grundelement unterhalb des Ofens angeordnet sein, und die Bewegungsvorrichtungen können aufrecht an einer Oberseite des Grundelements bewegbar sein.

In einem weiteren Ausführungsbeispiel weist der Ofen mindestens einen Heizkanal auf, der zu einer Längsaußenseite (z. B. Oberseite, Unterseite, linke Längsaußenseite oder rechte Längsaußenseite) hin offen ist. Das Grundelement ist an der Längsaußenseite angeordnet, vorzugsweise zum Bewegen der Behälterrohlinge mittels der mehreren Bewegungsvorrichtungen durch den Heizkanal.

In einem weiteren Ausführungsbeispiel weisen die mehreren Bewegungsvorrichtungen jeweils mindestens eine Halterung, vorzugsweise einen (z. B. passiven oder aktiven) Dorn, auf. Die mindestens eine Halterung ist dazu ausgebildet, mindestens einen Behälterrohling zu halten, vorzugsweise an einem Neckbereich des Behälterrohlings (z. B. innen oder außen an dem Neckbereich).

In einer Ausführungsform ist die mindestens eine Halterung wechselbar ausgeführt. Somit können vorteilhaft verschiedene Halterungen für unterschiedlich große und/oder unterschiedlich geformte Behälterrohlinge mit den Bewegungsvorrichtungen kombiniert werden.

In einer weiteren Ausführungsform weist die Vorrichtung ein Halterungsmagazin auf, das mehrere unterschiedliche Halterungen hält, wobei die mehreren Bewegungsvorrichtungen zu dem Halterungsmagazin zum, vorzugsweise automatischen, Wechseln der mindestens einen Halterung bewegbar sind. Vorteilhaft kann so ein Formatwechsel der Behälterrohlinge erheblich vereinfacht werden.

In einer weiteren Ausführungsform ist die mindestens eine Halterung mittels eines, vorzugsweise mechanischen, Drehmechanismus der jeweiligen Bewegungsvorrichtung zum Drehen des mindestens einen gehaltenen Behälterrohlings drehbar, vorzugsweise während des Bewegens durch den Ofen. Die Drehung kann vorzugsweise eine gleichmäßige oder ungleichmäßige Erwärmung der Behälterrohlinge ermöglichen. Es ist möglich, dass die Drehung gleichmäßig oder vorbestimmt ungleichmäßig ist. Eine vorbestimmt ungleichmäßige Drehung kann dazu eingesetzt werden, um gezielt wärmere und kältere Bereiche des Behälterrohlings zu erreichen (sogenanntes "preferential heating"). Damit können insbesondere Flaschen mit ungleichmäßigem Querschnitt, also z.B. quadratische oder ovale Flaschen, einfacher und besser erzeugt werden.

In einer Ausführungsvariante weist der Ofen eine Dreheinrichtung auf, die dazu ausgebildet ist, den Drehmechanismus, vorzugsweise mittels Formschluss und/oder Kraftschluss, zu drehen, während die jeweilige Bewegungsvorrichtung entlang der Dreheinrichtung bewegt wird. Bevorzugt kann so auf einen separaten Antrieb des Drehmechanismus verzichtet werden. Stattdessen kann der Drehmechanismus durch die Bewegung der Bewegungsvorrichtungen selbst angetrieben werden.

Bevorzugt kann sich die Dreheinrichtung zumindest abschnittsweise entlang eines Heizkanals des Ofens erstrecken.

In einer weiteren Ausführungsvariante weist der Drehmechanismus einen verzahnten Abschnitt, vorzugsweise ein Zahnrad, und die Dreheinrichtung einen verzahnten Abschnitt, vorzugsweise eine Zahnstange, auf, die zum Drehen des Drehmechanismus miteinander kämmen.

In einem Ausführungsbeispiel weist der Drehmechanismus eine Rolle und die Dreheinrichtung eine Abrollfläche für die Rolle auf.

In einem weiteren Ausführungsbeispiel wechselwirkt der Drehmechanismus mittels Magnetkraft mit der Dreheinrichtung zum Drehen des Drehmechanismus.

In einer Ausführungsform ist das Planarantriebssystem (z. B. mittels einer Steuereinheit) dazu ausgebildet, die mehreren Bewegungsvorrichtungen während des Bewegens der Behälterrohlinge durch den Ofen bezüglich des Grundelements um eine eigene Hochachse zu drehen, vorzugsweise zum gleichmäßigen oder ungleichmäßigen Erwärmen der Behälterrohlinge. Dies kann bspw. ermöglichen, auf einen separaten Drehmechanismus für die Halterung zu verzichten. Es ist möglich, dass die Drehung gleichmäßig oder vorbestimmt ungleichmäßig ist. Eine vorbestimmt ungleichmäßige Drehung kann dazu eingesetzt werden, um gezielt wärmere und kältere Bereiche des Behälterrohlings zu erreichen (sogenanntes "preferential heating").

In einer weiteren Ausführungsform ist das Planarantriebssystem (z. B. mittels einer Steuereinheit) dazu ausgebildet, bei einer Übernahme eines Behälterrohlings zu einer Bewegungsvorrichtung oder bei einer Übergabe eines Behälterrohlings von einer Bewegungsvorrichtung eine Hubbewegung der Bewegungsvorrichtung bezüglich des Grundelements durchzuführen, sodass vorzugsweise eine Halterung der Bewegungsvorrichtung in den Behälterrohling eintaucht oder aus dem Behälterrohling auftaucht.

In einer weiteren Ausführungsform ist das Planarantriebssystem (z. B. mittels einer Steuereinheit) dazu ausgebildet, einen in ungleichmäßigen Abständen an die mehreren Bewegungsvorrichtungen übergebenden Strom der Behälterrohlinge in einem gleichmäßigen Abstand durch den Ofen zu bewegen. Dadurch kann beispielsweise eine Ausschussrate aufgrund von zu starker Erwärmung einiger Behälterrohlinge verhindert werden, die durch zu große Lücken im Behälterrohlingstrom verursacht werden können.

In einer Ausführungsvariante ist das Planarantriebssystem (z. B. mittels einer Steuereinheit) dazu ausgebildet, eine Länge einer Heizstrecke der Bewegung der Behälterrohlinge durch den Ofen mittels der mehreren Bewegungsvorrichtungen anzupassen, vorzugsweise in Abhängigkeit von einem gewünschten Behälterrohlingdurchsatz.

In einer weiteren Ausführungsvariante ist das Planarantriebssystem (z. B. mittels einer Steuereinheit) dazu ausgebildet, eine Verweildauer der Behälterrohlinge im Ofen mittels Anpassen einer Geschwindigkeit der mehreren Bewegungsvorrichtungen anzupassen, vorzugsweise in Abhängigkeit von einem gewünschten Behälterrohlingdurchsatz.

In einem Ausführungsbeispiel weist der Ofen mehrere, vorzugweise individuell aktivierbare, Heizkanäle auf, durch die die Behälterrohlinge mittels der mehreren Bewegungsvorrichtungen flexibel bewegbar sind, vorzugsweise in Abhängigkeit von einer jeweils gewünschten Länge einer Heizstrecke der Bewegung der Behälterrohlinge durch den Ofen.

In einem weiteren Ausführungsbeispiel weist die Vorrichtung ferner eine Sterilisationseinrichtung zum Sterilisieren der Behälterrohlinge, vorzugsweise mittels Wasserstoffperoxid oder elektromagnetischer Bestrahlung, auf. Die Behälterrohlinge sind vorzugsweise mittels der mehreren Bewegungsvorrichtungen durch die Sterilisationseinrichtung bewegbar, vorzugsweise stromabwärts des Ofens. Vorteilhaft kann so eine besonders hygienische Behälterherstellung ermöglicht werden. Es ist auch möglich, dass die Sterilisationseinrichtung ein Abschnitt des Ofens ist, vorzugsweise an einem Auslassabschnitt des Ofens.

In einem weiteren Ausführungsbeispiel weist die Vorrichtung ferner eine Blasmaschine, vorzugsweise Streckblasmaschine, zum Blasen von Behältern aus den Behälterrohlingen auf, wobei die Blasmaschine stromabwärts des Ofens angeordnet und/oder zum Übernehmen von Behälterrohlingen von den mehreren Bewegungsvorrichtungen ausgebildet ist. Die Übernahme kann beispielsweise direkt erfolgen, oder es kann beispielsweise ein Transferstern (z. B. ein Teilungsverzugsstern) zwischen dem Grundelement und der Blasmaschine zur Übernahme der Behälterrohlinge angeordnet sein.

Vorzugsweise kann sich der Begriff "Steuereinheit" auf eine Elektronik (z. B. mit Mikroprozessor(en) und Datenspeicher) beziehen, die je nach Ausbildung Steuerungsaufgaben und/oder Regelungsaufgaben und/oder Verarbeitungsaufgaben übernehmen kann. Auch wenn hierin der Begriff "Steuern" verwendet wird, kann damit gleichsam zweckmäßig auch "Regeln" bzw. "Steuern mit Rückkopplung" und/oder "Verarbeiten" umfasst bzw. gemeint sein.

Die zuvor beschriebenen bevorzugten Ausführungsformen und Merkmale der Erfindung sind beliebig miteinander kombinierbar.

### Kurzbeschreibung der Figuren

Weitere Einzelheiten und Vorteile der Erfindung werden im Folgenden unter Bezug auf die beigefügten Zeichnungen beschrieben. Es zeigen:
- Figur 1: ein schematisches Blockschaltbild einer Vorrichtung zum Behandeln von Behälterrohlingen;
- Figur 2: ein schematisches Blockschaltbild einer weiteren Vorrichtung zum Behandeln von Behälterrohlingen;
- Figur 3: ein schematisches Blockschaltbild einer weiteren anderen Vorrichtung zum Behandeln von Behälterrohlingen;
- Figur 4: ein schematisches Blockschaltbild einer weiteren anderen Vorrichtung zum Behandeln von Behälterrohlingen;
- Figur 5: eine schematische Draufsicht auf eine weitere andere Vorrichtung zum Behandeln von Behälterrohlingen;
- Figur 6: eine schematische Seitenansicht eines Abschnitts eines Planarantriebssystems; und
- Figur 7: eine schematische Ansicht von unten auf einen Abschnitt eines Planarantriebssystems.

Die in den Figuren gezeigten Ausführungsformen stimmen zumindest teilweise überein, so dass ähnliche oder identische Teile mit den gleichen Bezugszeichen versehen sind und zu deren Erläuterung auch auf die Beschreibung der anderen Ausführungsformen bzw. Figuren verwiesen wird, um Wiederholungen zu vermeiden.

### Detaillierte Beschreibung beispielhafter Ausführungsformen

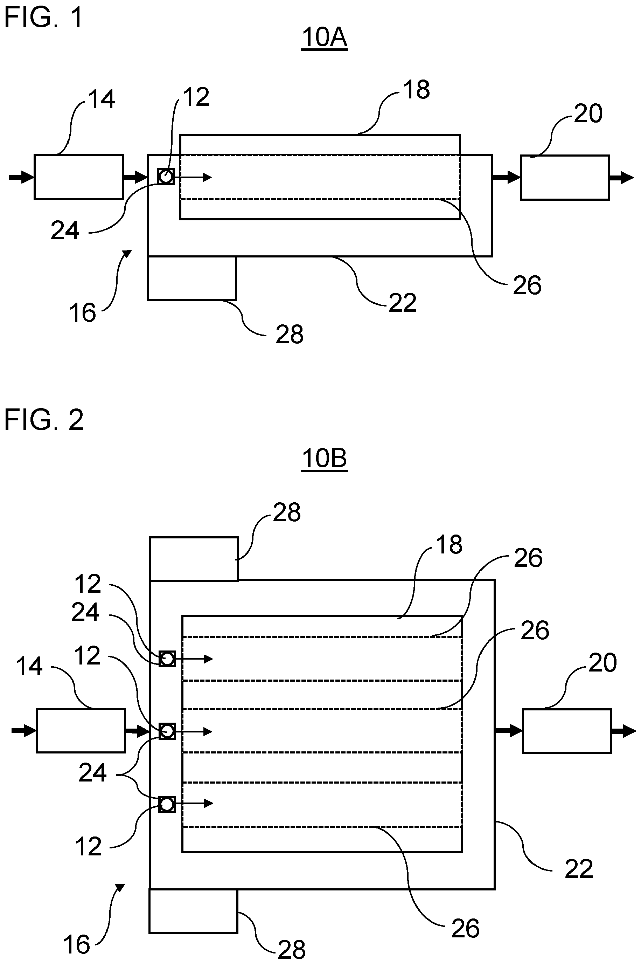

Figur 1 zeigt eine Vorrichtung 10A zur Behandlung von Behälterrohlingen 12. Aus Übersichtsgründen ist in Figur 1 nur ein Behälterrohling 12 dargestellt. Die Vorrichtung 10A weist einen Zulaufförderer 14, ein Planarantriebssystem 16, einen Ofen 18 und eine Blasmaschine 20 auf.

Der Zulaufförderer 14 fördert Behälterrohlinge 12 zu dem Planarantriebssystem 16. Beispielsweise kann ein Transferstern, z. B. ein Sägezahnstern, des Zulaufförderers 14 die Behälterrohlinge 12 an das Planarantriebssystem 16 übergeben. (Behälterrohling-) Stromaufwärts von dem Zulaufförderer 14 können weitere Einrichtungen angeordnet sein, z. B. eine Inspektionseinrichtung, die fehlerhafte Behälterrohlinge 12 aus dem Behälterrohlingstrom ausschleusen kann, und eine Sortiereinrichtung, die die Behälterrohlinge 12 in einer jeweils gewünschten Ausrichtung vereinzelt in einem Behälterrohlingstrom zur Verfügung stellen kann. Sofern eine Inspektionseinrichtung vorhanden ist, kann es im Betrieb vorkommen, dass der zu dem Planarantriebssystem 16 zugeführte Behälterstrom ungleichmäßige Abstände bzw. Lücken zwischen den nacheinander übergebenen Behälterrohlingen 12 aufweist. Die ungleichmäßigen Abstände können durch das Ausschleusen von fehlerhaften Behälterrohlingen 12 verursacht werden.

Das Planarantriebssystem 16 ist stromabwärts des Zulaufförderers 14 angeordnet. Das Planarantriebssystem 16 ist vorzugsweise ein magnetisches Planarantriebssystem.

Das Planarantriebssystem 16 weist ein Grundelement 22, mehrere Bewegungsvorrichtungen 24 (Mover bzw. Läufer) und eine Steuereinheit auf. Aus Übersichtsgründen ist lediglich eine Bewegungsvorrichtung 24 und keine Steuereinheit in Figur 1 dargestellt.

Die Bewegungsvorrichtungen 24 können mittels magnetischer Wechselwirkung mit dem Grundelement 22 frei und unabhängig voneinander über das Grundelement 22 bewegt werden. Die Steuereinheit ist dazu ausgebildet, eine Bewegung der Bewegungsvorrichtungen 24 bezüglich des Grundelements 22 zu steuern.

Die Bewegungsvorrichtungen 24 können auch mittels magnetischer Wechselwirkung mit dem Grundelement 22 bezüglich des Grundelements 22 gedreht werden (z. B. Gieren). Die Bewegungsvorrichtungen 24 können auch mittels magnetischer Wechselwirkung mit dem Grundelement 22 bezüglich des Grundelements 22 geneigt werden (z. B. Nicken und/oder Rollen). Die Bewegungsvorrichtungen 24 können ebenfalls mittels magnetischer Wechselwirkung mit dem Grundelement 22 bezüglich des Grundelements 22 eine Hubbewegung nach oben oder nach unten ausführen. Eine Anzahl der Bewegungsvorrichtungen 24 kann je nach Anwendungsfall frei wählbar sein.

Das Grundelement 22 bildet einen Stator des Planarantriebssystems 16. Die Bewegungsvorrichtungen 24 bilden Läufer des Planarantriebssystems 16. Die Bewegungsvorrichtungen 24 werden berührungslos von dem Grundelement 22 getragen, vorzugsweise auf einer Oberseite oder einer Unterseite des Grundelements 22. Das Grundelement 22 kann beispielsweise mehrere, verteilt angeordnete Elektromagnete, z. B. elektrische Spulen, aufweisen. Die Bewegungsvorrichtungen 24 können Permanentmagnete aufweisen. Die Elektromagnete sind bevorzugt in einer Matrix angeordnet, welche sich in einer Ebene des Grundelements 22 erstreckt (hier bevorzugt Horizontalebene). Die Steuereinheit des Planarantriebssystems 16 kann eine Stromzuführung zu den Elektromagneten des Grundelements 22 steuern, um elektromagnetische Felder an einer gewünschten Position des Grundelements 22 mit einer gewünschten Feldstärke aufzubauen. Ein Vortrieb, eine Drehung, eine Neigung und/oder ein Hub der Bewegungsvorrichtungen 24 kann durch entsprechende elektromagnetische Felder der Elektromagneten des Grundelements 22 bewirkt werden. Bevorzugt ist das Grundelement 22 in einer Horizontalebene ausgerichtet. Allerdings sind auch andere Ausrichtungen denkbar, z. B. eine zur Horizontalebene geneigte Ausrichtung, vorzugsweise eine Vertikalausrichtung.

Das Grundelement 22 kann verschiedene Ausprägungen und Formen haben, z. B. streifenförmig, rechteckig, quadratisch, vieleckig, rund, kreisrund usw. Für Reinraumanwendungen kann das Grundelement 22 zumindest teilweise innerhalb eines Reinraumes angeordnet sein oder eine, z. B. untere, Begrenzungswand des Reinraums bilden.

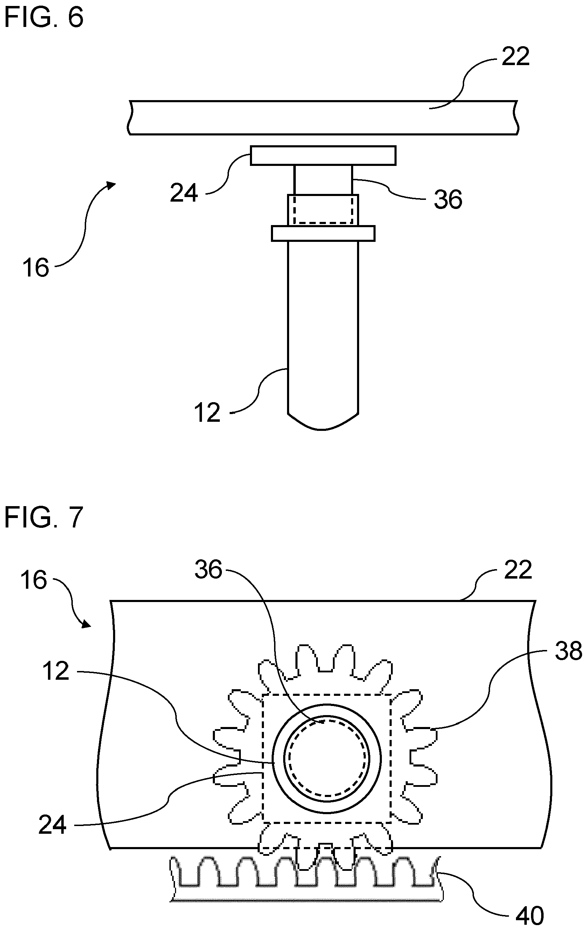

Die Bewegungsvorrichtungen 24 sind dazu ausgebildet, die Behälterrohlinge 12 zu bewegen, wenn sich die jeweilige Bewegungsvorrichtung 24 relativ zum Grundelement 16 bewegt. Dafür können die Bewegungsvorrichtungen 24 bspw. jeweils mindestens eine Halterung aufweisen, mit der jeweils mindestens ein Behälterrohling 12 gehalten werden kann. Hierin ist unter Bezugnahme auf Figur 6 ein Ausführungsbeispiel für eine Halterung beschrieben.

Die Behälterrohlinge 12 können durch den Zulaufförderer 14 (zum Beispiel Bandförderer oder Transferstern) zu dem Grundelement 22 bzw. den Bewegungsvorrichtungen 24 transportiert werden. Die Übernahme der Behälterrohlinge 12 zu den Bewegungsvorrichtungen 24 kann bspw. von einer Hubbewegung der jeweiligen Bewegungsvorrichtung 24 bezüglich des Grundelements 22 bewirkt oder unterstützt werden. Beispielsweise kann die jeweilige Bewegungsvorrichtung 24 sich zumindest temporär von dem Grundelement 22 entfernen, um zu ermöglichen, dass eine Halterung der Bewegungsvorrichtung 24 in den Behälterrohling 12 aufgrund der Hubbewegung eindringt bzw. eintaucht. Der Behälterrohling 12 kann so von dem Zulaufförderer 14 übernommen werden. Nach der Übernahme des Behälterrohlings 12 kann die Bewegungsvorrichtung 24 sich wieder mit einer Hubbewegung an das Grundelement 22 annähern.

Der (Heiz-) Ofen 18 ist dazu ausgebildet, die Behälterrohlinge 12 zu erwärmen. Der Ofen 18 und das Planarantriebssystem 16 sind so zueinander angeordnet, dass die Bewegungsvorrichtungen 24 die Behälterrohlinge 12 durch den Ofen 18 bewegen können.

Bevorzugt kann der Ofen 18 mindestens einen Heizkanal 26 zum Erwärmen der Behälterrohlinge 12 aufweisen. Der Heizkanal 26 kann geradlinig verlaufen oder einen geschwungenen Verlauf aufweisen. Der Heizkanal 26 kann an mindestens einer Längsseite mit Heizsegmenten, z. B. Infrarotheizelementen, elektrischen Heizplatten, Mikrowellen-Heizer oder Laser-Heizer, ausgestattet sein. Es ist möglich, dass der Heizkanal 26 auch Reflektoren auf einer den Heizsegmenten gegenüberliegenden Seite des Heizkanals 26 aufweist.

Der Heizkanale 26 kann zu einer Längsaußenseite (z. B. Oberseite, Unterseite, linke Außenseite, rechte Außenseite) hin offen ist. Das Grundelement 22 ist an der offenen Längsaußenseite des Heizkanals 26 angeordnet. Die Behälterrohlinge 12 können von den Bewegungsvorrichtungen 24 durch die offene Längsaußenseite hindurch durch den Heizkanal 26 bewegt werden.

Beispielsweise kann der Heizkanal 26 an einer Oberseite offen sein. Das Grundelement 22 kann oberhalb des Heizkanals 26 angeordnet sein. Die Bewegungsvorrichtungen 24 können über Kopf an einer Unterseite des Grundelements 22 bewegbar sein. Die Bewegungsvorrichtungen 24 können sich außerhalb des Heizkanals 26 an dem Grundelement 22 bewegen und dabei die Behälterrohlinge 12 durch die offene Oberseite hindurch durch den Heizkanal 26 bewegen.

Alternativ kann der Heizkanal 26 bspw. an einer Unterseite offen sein. Das Grundelement 22 kann unterhalb des Heizkanals 26 angeordnet sein. Die Bewegungsvorrichtungen 24 können aufrecht an einer Oberseite des Grundelements 22 bewegbar sein. Die Bewegungsvorrichtungen 24 können sich außerhalb des Heizkanals 26 an dem Grundelement 22 bewegen und dabei die Behälterrohlinge 12 durch die offene Unterseite hindurch durch den Heizkanal 26 bewegen.

Während die Bewegungsvorrichtungen 24 sich mit den Behälterrohlingen 12 durch den Ofen 18 bewegen, können die Behälterrohlinge 12 gedreht werden. So kann vorzugsweise eine gleichmäßige Erwärmung der Behälterrohlinge 12 erzielt werden. Die Drehung kann durch einen Drehmechanismus der Bewegungsvorrichtung 24 bewirkt werden (siehe z. B. Ausführungsbeispiel von Figur 7). Die Drehung kann auch durch eine Drehung der Bewegungsvorrichtungen 24 bewirkt werden, die sich bezüglich des Grundelements 22 um eine eigene Hochachse drehen können, z. B. während sie sich durch den mindestens einen Heizkanal 26 bewegen. Es ist auch möglich, dass sich die Drehung mittels des Drehmechanismus und die Drehung mittels der jeweiligen Bewegungsvorrichtung 24 überlagern, z. B. um abschnittsweise die durch den Drehmechanismus bewirkte Drehung durch die mittels der Bewegungsvorrichtung 24 bewirkte Drehung zumindest teilweise auszugleichen / zu kompensieren. So kann bspw. eine optimale Temperaturverteilung gewährleistet werden, insbesondere bei Formflaschen / sogenannten "shaped bottles" usw.

Die Bewegungsvorrichtungen 24 bewegen sich in gleichen Abständen durch den Ofen 18. Sofern der Zulaufförderer 14 einen zumindest abschnittsweise ungleichmäßigen Strom an Behälterrohlingen 12 an die Bewegungsvorrichtungen 24 übergibt, können die Bewegungsvorrichtungen 24 so zueinander bewegt werden (z. B. Beschleunigen, Verzögern, Abstände variieren usw.), dass wieder im Wesentlichen gleiche Abstände zwischen den Behälterrohlingen 12, die von den Bewegungsvorrichtungen 24 transportiert werden, vorliegen.

Es ist möglich, dass ein oder mehrere Abfuhrförderer (zum Beispiel Bandförderer oder Transportstern) angeordnet sind, um die vom Ofen 18 erwärmten Behälterrohlinge 12 von den Bewegungsvorrichtungen 24 zu übernehmen und zu der Blasmaschine 20 zu transportieren. Auch bei der Übergabe zum Abfuhrförderer kann die jeweilige Bewegungsvorrichtung 24 eine Hubbewegung bezüglich des Grundelements 22 ausführen, um die Übergabe zu bewirken oder zu unterstützen, wenn gewünscht. Durch die Hubbewegung kann die Halterung bspw. aus dem erwärmten Behälterrohling 12 auftauchen bzw. entfernt werden, z. B. während der erwärmte Rohling bereits von dem Abfuhrförderer anderweitig gehalten ist.

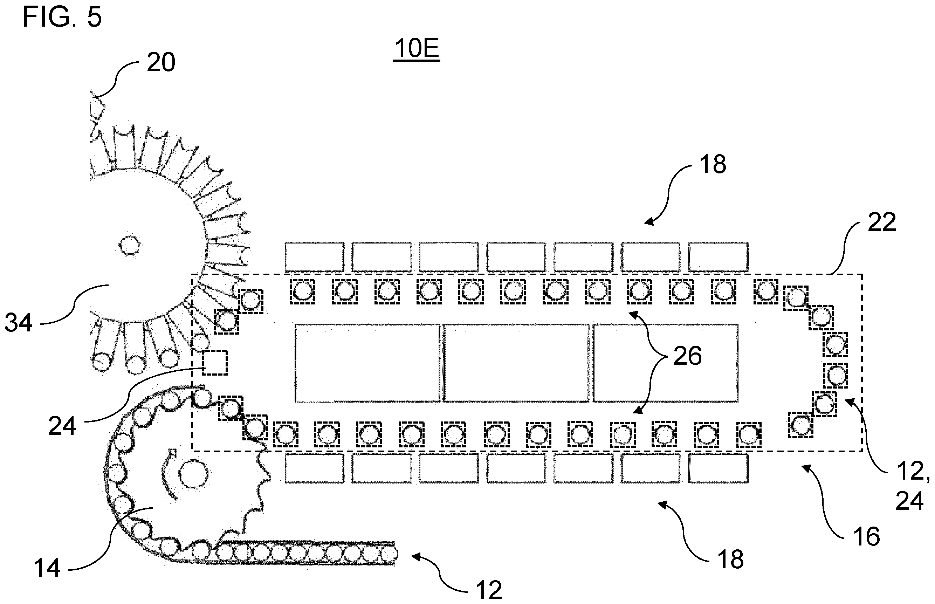

Nach der Übergabe des erwärmten Behälterrohlings 12 kann die jeweilige Bewegungsvorrichtung 24 wieder zurück zum Zulaufförderer 14 bewegt werden. Beim Zulaufförderer 14 kann ein weiterer Behälterrohling 12 übernommen werden. Vorzugsweise umfährt die Bewegungsvorrichtung 24 auf dem Rückweg zum Zulaufförderer 14 den Ofen 18. Beispielsweise kann die Bewegungsvorrichtung 24 neben dem Ofen 18 entlang des Grundelements 22, z. B. entlang eines Randbereichs des Grundelements 22, zurückbewegt werden. Es ist auch möglich, dass ein Einlass und ein Auslass des Ofens 18 nebeneinander angeordnet sind (vgl. z. B. Ausführungsbeispiel von Figur 5), was den Rückweg deutlich verkürzen kann.

Die Blasmaschine 20 ist stromabwärts von dem Ofen 18 und dem Planarantriebssystem 16 angeordnet. In der Blasmaschine 20 können die vom Ofen 18 erwärmten Behälterrohlinge 12 zu Behältern geblasen werden. Die Blasmaschine 20 kann beispielsweise als eine Streckblasmaschine, z. B. in Karussellform, ausgeführt sein. Alternativ kann die Blasmaschine 20 beispielsweise stationär angeordnet und/oder modular aufgebaut sein. Stromabwärts der Blasmaschine 20 können beispielsweise noch ein Füller (z. B. Füllerkarussell) zum Befüllen der Behälter und ein Verschließer zum Verschließen der Behälter angeordnet sein oder auch eine oder mehrere Beschichtungsstationen zur Innenbeschichtung der Behälterrohlinge 12 oder Behälter vorgesehen sein.

Optional kann die Vorrichtung 10A ein Halterungsmagazin 28 aufweisen. Das Halterungsmagazin 28 kann in Reichweite der Bewegungsvorrichtungen 24 angeordnet sein, z. B. an einem Rand des Grundelements 22. Das Halterungsmagazin 28 kann mehrere unterschiedliche Halterungen für unterschiedlich dimensionierte Behälterrohlinge 12 bevorraten. Beispielsweise können die Halterungen an unterschiedliche Neck- / Hals- / Mundstückgeometrien der Behälterrohlinge 12 angepasst sein. Für einen Formatwechsel können die Bewegungsvorrichtungen 24 zu dem Halterungsmagazin 28 bewegt werden. Beim Halterungsmagazin 28 kann die aktuelle Halterung der jeweiligen Bewegungsvorrichtung 24 durch eine andere Halterung automatisch ausgetauscht werden. Die Halterung kann bevorzugt mittels eines Schnellwechselsystems, vorzugsweise eines Steck-, Rast- und/oder Schraub-Schnellwechselsystems, an der jeweiligen Bewegungsvorrichtung 24 angebracht sein bzw. gewechselt werden. Vorzugsweise weist das Halterungsmagazin 28 jeweils mehrere Halterungen je Halterungstyp auf, sodass mehrere Bewegungsvorrichtungen 24 die Halterung wechseln können.

Figur 2 zeigt eine modifizierte Vorrichtung 10B.

Bei der Vorrichtung 10B weist der Ofen 18 mehrere Heizkanäle 26 auf. Die Heizkanäle 26 sind beispielsweise parallel zueinander angeordnet. Je nach Durchsatz können die Heizkanäle 26 zugeschaltet oder abgeschaltet werden. Beispielsweise können bei einem Maximaldurchsatz alle Heizkanäle 26 aktiviert sein. Durch alle Heizkanäle 26 werden die Behälterrohlinge 12 mit den Bewegungsvorrichtungen 24 transportiert. Bei einem Minimaldurchsatz ist beispielsweise nur einer der mehreren Heizkanäle 26 aktiviert. Nur durch den aktivierten Heizkanal 26 werden die Behälterrohlinge 12 mit den Bewegungsvorrichtungen 24 transportiert. Die übrigen (inaktiven) Heizkanäle bleiben ungenutzt oder können von den Bewegungsvorrichtungen 24 auf dem Rückweg zum Zulaufförderer 14 genutzt werden. Es wäre auch möglich, mehrere stationäre Blasstationen bzw. Blasmaschinen 20 hinter den einzelnen Heizkanälen anzuordnen.

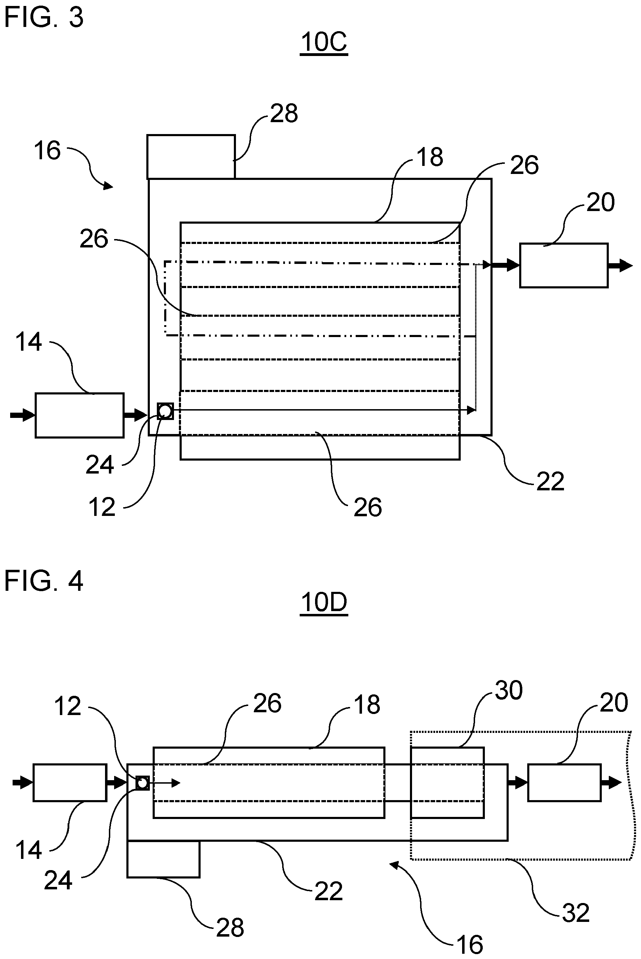

Figur 3 zeigt eine modifizierte Vorrichtung 10C.

Die Vorrichtung 10C unterscheidet sich von der Vorrichtung 10B dadurch, dass die Bewegungsvorrichtungen 24 unterschiedlich lange Heizstrecken durch den Ofen 18 zurücklegen können. Bspw. kann eine kurze Heizstrecke bei einem kleinen Durchsatz gewählt werden. Die kurze Heizstrecke führt nur durch einen Heizkanal 26. Bei einem großen Durchsatz kann hingegen eine lange Heizstrecke gewählt werden, z. B. aufweisend zwei oder mehr Heizkanäle. Eine Länge der Heizstrecke durch den Ofen 18 kann somit durch die Bewegungsvorrichtungen 24 angepasst werden, vorzugsweise in Abhängigkeit von einem aktuellen Durchsatz. Mit steigendem Durchsatz kann eine Heizstrecke durch den Ofen 18 verlängert werden. Variable Längen der Heizstrecke durch den Ofen 18 können alternativ oder zusätzlich auch durch mehrere Ausgänge entlang eines Heizkanals 26 bereitgestellt werden. Es ist auch möglich, dass eine Bewegungsgeschwindigkeit der Bewegungsvorrichtungen 24 durch den Ofen 18 anpassbar ist, um eine gewünschte Verweildauer im Ofen 18 anzupassen.

Figur 4 zeigt eine modifizierte Vorrichtung 10D.

Die Vorrichtung 10D weist eine Sterilisationseinrichtung 30 auf. Die Sterilisationseinrichtung 30 ist stromabwärts des Ofens 18 angeordnet. Die Sterilisationseinrichtung 30 ist stromaufwärts der Blasmaschine 20 angeordnet. Die Sterilisationseinrichtung 30 ist mit dem Planarantriebssystem 16 verbunden. Die Bewegungsvorrichtungen 24 können mit den transportierten Behälterrohlingen 12 durch die Sterilisationseinrichtung 30 bewegt werden. In der Sterilisationseinrichtung 30 können die Behälterrohlinge 12 sterilisiert werden. Beispielsweise können die Behälterrohlinge 12 mit Wasserstoffperoxid oder mit elektromagnetischer Strahlung behandelt werden.

Die Vorrichtung 10D kann einen Reinraum 32 aufweisen. In dem Reinraum 32 können u.a. die Sterilisationseinrichtung 30 und die Blasmaschine 20 angeordnet sein (sowie z. B. ein Füller und ein Verschließer). Im Reinraum kann bspw. ein (leichter) Überdruck herrschen, um ein Eindringen von Partikeln in der Luft zu verhindern. Der Reinraum 32 kann mit dem Planarantriebssystem 16 verbunden sein. Die Bewegungsvorrichtungen 24 können in den Reinraum 32 einfahren und aus dem Reinraum 32 ausfahren, z. B. durch mindestens eine Schleuse.

Figur 5 zeigt eine Vorrichtung 10E, die mehr strukturelle Details offenbart als die Vorrichtungen 10A bis 10D.

Die Vorrichtung 10E weist einen Ofen 18 mit U-förmigem Heizkanal 26 auf. Über dem Ofen 18 ist das Grundelement 22 angeordnet (transparent dargestellt). Die Bewegungsvorrichtungen 24 (ebenfalls transparent dargestellt) bewegen sich über Kopf an einer Unterseite des Grundelements 22. Ein als Sägezahnstern ausgeführter Zulaufförderer 14 übergibt die Behälterrohlinge 12 an die Bewegungsvorrichtungen 24. Die Bewegungsvorrichtungen 24 bewegen die Behälterrohlinge 12 durch den Heizkanal 26. Die Bewegungsvorrichtungen 24 übergeben die erwärmten Behälterrohlinge 12 an einen als Transportstern ausgeführten Abfuhrförderer 34, der die Behälterrohlinge 12 an die Blasmaschine 20 (nur abschnittsweise dargestellt) übergibt.

Figur 6 zeigt eine beispielhafte Bewegungsvorrichtung 24.

Die Bewegungsvorrichtung 24 ist über Kopf an einer Unterseite (Antriebsfläche) des Grundelements 22 bewegbar. Die Bewegungsvorrichtung 24 ist, vorzugsweise berührungslos, an dem Grundelement 22 getragen. Die Bewegungsvorrichtung 24 trägt eine Halterung 36 für die Behälterrohlinge 12. Die Halterung 36 ist bevorzugt als ein aktiver oder passiver Dorn ausgeführt. Die Halterung 36 kann in eine Öffnung eines Halsbereichs bzw. Neckbereichs des Behälterrohlings 12 eingeführt werden, z. B. mittels Hubbewegung der Bewegungsvorrichtung 24 nach unten relativ zum Grundelement 22. Die Halterung 36 kann sich innen im Halsbereich mehrseitig abstützen und so den Behälterrohling 12 halten. Es ist auch möglich, dass die Halterung 36 anders ausgeführt ist, und z. B. den Halsbereich umgreifen kann.

Figur 7 zeigt eine Variante der Bewegungsvorrichtung 24 mit einem Drehmechanismus 38.

Der Drehmechanismus 38 kann dazu verwendet werden, den oder die gehaltenen Behälterrohlinge 12 im Ofen 18 zu drehen. Der Drehmechanismus 38 ermöglicht eine Drehung der Halterung 36 und damit des Behälterrohlings 12, ohne dass sich hierfür die Bewegungsvorrichtung 24 dreht. Der Drehmechanismus 38 tritt dazu in Wechselwirkung mit einer Dreheinrichtung 40. Die Dreheinrichtung 40 kann ein Teil des Ofens 18 sein. Die Dreheinrichtung 48 kann sich zumindest abschnittsweise entlang des Heizkanals 26 des Ofens 18 erstrecken (siehe z. B. Figur 1).

Der Drehmechanismus 38 lagert die Halterung 36 drehbar auf einer Basis der Bewegungsvorrichtung 24. Der Drehmechanismus 38 kann beispielsweise ein außenverzahnter Drehteller (zum Beispiel Zahnrad) sein, auf dem die Halterung 36 angebracht ist. Die Dreheinrichtung 40 ist stationär und derart ausgebildet, dass sie mit dem Drehmechanismus 38 zum Drehen des Drehmechanismus 38 in Wechselwirkung treten kann, wenn die Bewegungsvorrichtung 24 nahe genug entlang der Dreheinrichtung 40 bewegt wird. Beispielsweise kann die Dreheinrichtung 40 eine Zahnstange sein. Die Zahnstange kann beispielsweise gerade, wie in Figur 7 dargestellt ist, oder gebogen sein. Der Drehmechanismus 38 kann mit der Dreheinrichtung 40 zum Drehen des Drehmechanismus 38 und somit der Halterung 36 kämmen.

Es ist möglich, dass der Drehmechanismus 38 und die Dreheinrichtung 40 anders ausgeführt sind. Beispielsweise könnte der Drehmechanismus 38 als ein Rad oder einer Rolle ausgeführt sein, und die Dreheinrichtung 40 könnte als eine Abrollfläche ausgeführt sein. In einem anderen Beispiel ist ein Außenumfang des als Drehteller ausgeführten Drehmechanismus 38 aus einem (ferro-) magnetischen Material hergestellt, und die Dreheinrichtung 40 weist mindestens einen Permanentmagnet auf, oder umgekehrt. Prinzipiell kann der Drehmechanismus 38 vorzugsweise mittels Kraftschluss und/oder mittels Formschluss von der Dreheinrichtung 40 gedreht werden, wenn sich die Bewegungsvorrichtung 24 entlang der Dreheinrichtung 40 bewegt.

### Bezugszeichenliste

- 10A-10E: Vorrichtung zur Behandlung von Behälterrohlingen
- 12: Behälterrohling
- 14: Zulaufförderer
- 16: Planarantriebssystem
- 18: Ofen
- 20: Blasmaschine
- 22: Grundelement
- 24: Bewegungsvorrichtung
- 26: Heizkanal
- 28: Halterungsmagazin
- 30: Sterilisationseinrichtung
- 32: Reinraum
- 34: Abfuhrförderer
- 36: Halterung
- 38: Drehmechanismus
- 40: Dreheinrichtung

## Patentansprüche

1. Vorrichtung (10A-10E) zur Behandlung von Behälterrohlingen (12) für eine Behälterbehandlungsanlage, aufweisend:
einen Ofen (18) zum Erwärmen der Behälterrohlinge (12); und
ein Planarantriebssystem (16) aufweisend ein Grundelement (22) und mehrere Bewegungsvorrichtungen (24) zum Transportieren der Behälterrohlinge (12),
wobei die mehreren Bewegungsvorrichtungen (24) bezüglich des Grundelements (22) unabhängig voneinander bewegbar sind, vorzugsweise mittels magnetischer Wechselwirkung zwischen dem Grundelement (22) und den mehreren Bewegungsvorrichtungen (24), und
wobei die Behälterrohlinge (12) mittels der mehreren Bewegungsvorrichtungen (24) durch den Ofen (18) bewegbar sind.

2. Vorrichtung (10A-10E) nach Anspruch 1, wobei:
das Grundelement (22) oberhalb des Ofens (18) angeordnet ist und die mehreren Bewegungsvorrichtungen (24) über Kopf an einer Unterseite des Grundelements (22) bewegbar sind; oder
das Grundelement (22) unterhalb des Ofens (18) angeordnet ist und die mehreren Bewegungsvorrichtungen (24) aufrecht an einer Oberseite des Grundelements (22) bewegbar sind.

3. Vorrichtung (10A-10E) nach Anspruch 1 oder Anspruch 2, wobei:
der Ofen (18) mindestens einen Heizkanal (26) aufweist, der zu einer Längsaußenseite hin offen ist; und
das Grundelement (22) an der Längsaußenseite angeordnet ist, vorzugsweise zum Bewegen der Behälterrohlinge (12) mittels der mehreren Bewegungsvorrichtungen (24) durch den Heizkanal (26).

4. Vorrichtung (10A-10E) nach einem der vorherigen Ansprüche, wobei:
die mehreren Bewegungsvorrichtungen (24) jeweils mindestens eine Halterung (36), vorzugsweise einen Dorn, aufweisen, die dazu ausgebildet ist, mindestens einen Behälterrohling (12) zu halten, vorzugsweise an einem Neckbereich des Behälterrohlings (12).

5. Vorrichtung (10A-10E) nach Anspruch 4, wobei:
die mindestens eine Halterung (36) wechselbar ausgeführt ist; und/oder
die Vorrichtung ein Halterungsmagazin (28) aufweist, das mehrere unterschiedliche Halterungen (36) hält, wobei die mehreren Bewegungsvorrichtungen (24) zu dem Halterungsmagazin (28) zum, vorzugsweise automatischen, Wechseln der mindestens einen Halterung (36) bewegbar sind.

6. Vorrichtung (10A-10E) nach Anspruch 4 oder Anspruch 5, wobei:
die mindestens eine Halterung (36) mittels eines, vorzugsweise mechanischen, Drehmechanismus (38) der jeweiligen Bewegungsvorrichtung (24) zum Drehen des mindestens einen gehaltenen Behälterrohlings (12) drehbar ist, vorzugsweise während des Bewegens durch den Ofen (18).

7. Vorrichtung (10A-10E) nach Anspruch 6, wobei:
der Ofen (18) eine Dreheinrichtung (40) aufweist, die dazu ausgebildet ist, den Drehmechanismus (38), vorzugsweise mittels Formschluss und/oder Kraftschluss, zu drehen, während die jeweilige Bewegungsvorrichtung (24) entlang der Dreheinrichtung (40) bewegt wird.

8. Vorrichtung (10A-10E) nach Anspruch 7, wobei:
der Drehmechanismus (38) einen verzahnten Abschnitt, vorzugsweise ein Zahnrad, und die Dreheinrichtung (40) einen verzahnten Abschnitt, vorzugsweise eine Zahnstange, aufweist, die zum Drehen des Drehmechanismus miteinander kämmen; oder
der Drehmechanismus (38) eine Rolle und die Dreheinrichtung (40) eine Abrollfläche für die Rolle aufweist; oder
der Drehmechanismus (38) mittels Magnetkraft mit der Dreheinrichtung (40) zum Drehen des Drehmechanismus (38) wechselwirkt.

9. Vorrichtung (10A-10E) nach einem der vorherigen Ansprüche, wobei:
das Planarantriebssystem (16) dazu ausgebildet ist, die mehreren Bewegungsvorrichtungen (24) während des Bewegens der Behälterrohlinge (12) durch den Ofen (18) bezüglich des Grundelements (22) um eine eigene Hochachse zu drehen, vorzugsweise zum gleichmäßigen Erwärmen der Behälterrohlinge (12).

10. Vorrichtung (10A-10E) nach einem der vorherigen Ansprüche, wobei:
das Planarantriebssystem (16) dazu ausgebildet ist, bei einer Übernahme eines Behälterrohlings (12) zu einer Bewegungsvorrichtung (24) oder bei einer Übergabe eines Behälterrohlings (12) von einer Bewegungsvorrichtung (24) eine Hubbewegung der Bewegungsvorrichtung (24) bezüglich des Grundelements (22) durchzuführen, sodass vorzugsweise eine Halterung (36) der Bewegungsvorrichtung (24) in den Behälterrohling (12) eintaucht oder aus dem Behälterrohling (12) auftaucht.

11. Vorrichtung (10A-10E) nach einem der vorherigen Ansprüche, wobei:
das Planarantriebssystem (16) dazu ausgebildet ist, einen in ungleichmäßigen Abständen an die mehreren Bewegungsvorrichtungen (24) übergebenden Strom der Behälterrohlinge (12) in einem gleichmäßigen Abstand durch den Ofen (18) zu bewegen.

12. Vorrichtung (10A-10E) nach einem der vorherigen Ansprüche, wobei:
das Planarantriebssystem (16) dazu ausgebildet ist, eine Länge einer Heizstrecke der Bewegung der Behälterrohlinge (12) durch den Ofen (18) mittels der mehreren Bewegungsvorrichtungen (24) anzupassen, vorzugsweise in Abhängigkeit von einem gewünschten Behälterrohlingdurchsatz; und/oder
das Planarantriebssystem (16) dazu ausgebildet ist, eine Verweildauer der Behälterrohlinge (12) im Ofen (18) mittels Anpassen einer Geschwindigkeit der mehreren Bewegungsvorrichtungen (24) anzupassen, vorzugsweise in Abhängigkeit von einem gewünschten Behälterrohlingdurchsatz.

13. Vorrichtung (10B; 10C) nach einem der vorherigen Ansprüche, wobei:
der Ofen (18) mehrere, vorzugweise individuell aktivierbare, Heizkanäle (26) aufweist, durch die die Behälterrohlinge (12) mittels der mehreren Bewegungsvorrichtungen (24) flexibel bewegbar sind, vorzugsweise in Abhängigkeit von einer jeweils gewünschten Länge einer Heizstrecke der Bewegung der Behälterrohlinge (12) durch den Ofen (18).

14. Vorrichtung (10D) nach einem der vorherigen Ansprüche, ferner aufweisend:
eine Sterilisationseinrichtung (30) zum Sterilisieren der Behälterrohlinge (12), vorzugsweise mittels Wasserstoffperoxid oder elektromagnetischer Bestrahlung,
wobei die Behälterrohlinge (12) mittels der mehreren Bewegungsvorrichtungen (24) durch die Sterilisationseinrichtung (30) bewegbar sind, vorzugsweise stromabwärts des Ofens (18).

15. Vorrichtung (10A-10E) nach einem der vorherigen Ansprüche, ferner aufweisend:
eine Blasmaschine (20), vorzugsweise Streckblasmaschine, zum Blasen von Behältern aus den Behälterrohlingen (12), wobei die Blasmaschine (20) stromabwärts des Ofens (18) angeordnet und/oder zum Übernehmen von Behälterrohlingen (12) von den mehreren Bewegungsvorrichtungen (24) ausgebildet ist.

## Claims

1. A device (10A-10E) for treating container blanks (12) for a container treatment installation, having:
a furnace (18) for heating the container blanks (12); and
a planar drive system (16) having a base element (22) and a plurality of movement devices (24) for transporting the container blanks (12),
wherein the plurality of movement devices (24) are movable independently of one another relative to the base element (22), preferably by means of magnetic interaction between the base element (22) and the plurality of movement devices (24), and
wherein the container blanks (12) can be moved through the furnace (18) by means of the plurality of movement devices (24).

2. The device (10A-10E) as clamed in claim 1, wherein:
the base element (22) is arranged above the furnace (18), and the plurality of movement devices (24) can be moved upside down on an underside of the base element (22); or
the base element (22) is arranged beneath the furnace (18), and the plurality of movement devices (24) can be moved upright on an upper side of the base element (22).

3. The device (10A-10E) as claimed in claim 1 or claim 2, wherein:
the furnace (18) has at least one heating channel (26) which is open toward a longitudinal outer side; and
the base element (22) is arranged on the longitudinal outer side, preferably for moving the container blanks (12) through the heating channel (26) by means of the plurality of movement devices (24).

4. The device (10A-10E) as claimed in one of the preceding claims, wherein:
the plurality of movement devices (24) each have at least one holder (36), preferably a mandrel, which is configured to hold at least one container blank (12), preferably at a neck region of the container blank (12).

5. The device (10A-10E) as claimed in claim 4, wherein:
the at least one holder (36) is designed to be changeable; and/or
the device has a holder magazine (28) which holds a plurality of different holders (36), wherein the plurality of movement devices (24) can be moved to the holder magazine (28) in order to change, preferably automatically, the at least one holder (36).

6. The device (10A-10E) as claimed in claim 4 or claim 5, wherein:
the at least one holder (36) is rotatable by means of a rotation mechanism (38), preferably a mechanical rotation mechanism, of the respective movement device (24) in order to rotate the at least one held container blank (12), preferably while it is being moved through the furnace (18).

7. The device (10A-10E) as claimed in claim 6, wherein:
the furnace (18) has a rotation apparatus (40) which is configured to rotate the rotation mechanism (38), preferably by means of interlocking engagement and/or force-based engagement, while the respective movement device (24) is being moved along the rotation apparatus (40).

8. The device (10A-10E) as claimed in claim 7, wherein:
the rotation mechanism (38) has a toothed portion, preferably a toothed wheel, and the rotation apparatus (40) has a toothed portion, preferably a toothed rack, which mesh with one another in order to rotate the rotation mechanism; or
the rotation mechanism (38) has a roller and the rotation apparatus (40) has a rolling surface for the roller; or
the rotation mechanism (38) interacts with the rotation apparatus (40) by means of magnetic force in order to rotate the rotation mechanism (38).

9. The device (10A-10E) as claimed in one of the preceding claims, wherein:
the planar drive system (16) is configured to rotate the plurality of movement devices (24) relative to the base element (22) about their own vertical axis while the container blanks (12) are being moved through the furnace (18), preferably in order to heat the container blanks (12) uniformly.

10. The device (10A-10E) as claimed in one of the preceding claims, wherein:
the planar drive system (16) is configured to carry out a stroke movement of the movement device (24) relative to the base element (22) when a container blank (12) is transferred to a movement device (23) or when a container blank (12) is transferred from a movement device (24), so that preferably a holder (36) of the movement device (24) enters the container blank (12) or emerges from the container blank (12).

11. The device (10A-10E) as claimed in one of the preceding claims, wherein:
the planar drive system (16) is configured to move a stream of container blanks (12) transferred at non-uniform distances to the plurality of movement devices (24) through the furnace (18) at a uniform distance.

12. The device (10A-10E) as claimed in one of the preceding claims, wherein:
the planar drive system (16) is configured to adapt a length of a heating section of the movement of the container blanks (12) through the furnace (18) by means of the plurality of movement devices (24), preferably in dependence on a desired container blank throughput; and/or
the planar drive system (16) is configured to adapt a residence time of the container blanks (12) in the furnace (18) by adapting a speed of the plurality of movement devices (24), preferably in dependence on a desired container blank throughput.

13. The device (10B; 10C) as claimed in one of the preceding claims, wherein:
the furnace (18) has a plurality of preferably individually activatable heating channels (26) through which the container blanks (12) are movable in a flexible manner by means of the plurality of movement devices (24), preferably in dependence on a respective desired length of a heating section of the movement of the container blanks (12) through the furnace (18).

14. The device (10D) as claimed in one of the preceding claims, further having:
a sterilization apparatus (30) for sterilizing the container blanks (12), preferably by means of hydrogen peroxide or electromagnetic radiation,
wherein the container blanks (12) can be moved through the sterilization apparatus (30) by means of the plurality of movement devices (24), preferably downstream of the furnace (18).

15. The device (10A-10E) as claimed in one of the preceding claims, further having:
a blow molding machine (20), preferably a stretch blow molding machine, for blow molding containers from the container blanks (12), wherein the blow molding machine (20) is arranged downstream of the furnace (18) and/or is configured for the transfer of container blanks (12) from the plurality of movement devices (24).

## Revendications

1. Dispositif (10A-10E) destiné au traitement d'ébauches de récipients (12) pour une installation de traitement de récipients, présentant :
un four (18) pour le chauffage des ébauches de récipients (12) ; et
un système d'entraînement plan (16) présentant un élément formant base (22) et plusieurs dispositifs de déplacement (24) pour le transport des ébauches de récipients (12),
dans lequel les dispositifs de déplacement (24) peuvent être déplacés indépendamment les uns des autres par rapport à l'élément formant base (22), de préférence au moyen d'une interaction magnétique entre l'élément formant base (22) et les dispositifs de déplacement (24), et
dans lequel les ébauches de récipients (12) peuvent être déplacées à travers le four (18) au moyen des dispositifs de déplacement (24).

2. Dispositif (10A-10E) selon la revendication 1, dans lequel :
l'élément formant base (22) est disposé au-dessus du four (18) et les dispositifs de déplacement (24) peuvent être déplacés en hauteur au niveau d'une face inférieure de l'élément formant base (22) ; ou
l'élément formant base (22) est disposé en dessous du four (18) et les dispositifs de déplacement (24) peuvent être déplacés verticalement au niveau d'une face supérieure de l'élément formant base (22).

3. Dispositif (10A-10E) selon la revendication 1 ou la revendication 2, dans lequel :
le four (18) présente au moins un canal de chauffage (26) qui est ouvert vers une face extérieure longitudinale ; et
l'élément formant base (22) est disposé au niveau de la face extérieure longitudinale, de préférence pour le déplacement des ébauches de récipients (12) à travers le canal de chauffage (26) au moyen des dispositifs de déplacement (24).

4. Dispositif (10A-10E) selon l'une des revendications précédentes, dans lequel :
les dispositifs de déplacement (24) présentent respectivement au moins un moyen de maintien (36), de préférence un mandrin, qui est réalisé pour maintenir au moins une ébauche de récipient (12), de préférence au niveau d'une zone de goulot de l'ébauche de récipient (12).

5. Dispositif (10A-10E) selon la revendication 4, dans lequel :
l'au moins un moyen de maintien (36) est conçu de manière à pouvoir être changé ; et/ou
le dispositif présente un magasin de moyens de maintien (28) qui maintient plusieurs moyens de maintien (36) différents, dans lequel les dispositifs de déplacement (24) peuvent être déplacés vers le magasin de moyens de maintien (28) pour le changement, de préférence automatique, de l'au moins un moyen de maintien (36).

6. Dispositif (10A-10E) selon la revendication 4 ou la revendication 5, dans lequel :
l'au moins un moyen de maintien (36) peut être tourné au moyen d'un mécanisme de rotation (38), de préférence mécanique, du dispositif de déplacement (24) respectif pour la rotation de l'au moins une ébauche de récipient (12) maintenue, de préférence pendant le déplacement à travers le four (18).

7. Dispositif (10A-10E) selon la revendication 6, dans lequel :
le four (18) présente un appareil de rotation (40) qui est réalisé pour faire tourner le mécanisme de rotation (38), de préférence au moyen d'une liaison par complémentarité de forme et/ou d'une liaison par adhérence, pendant que le dispositif de déplacement (24) respectif est déplacé le long de l'appareil de rotation (40).

8. Dispositif (10A-10E) selon la revendication 7, dans lequel :
le mécanisme de rotation (38) présente une section dentée, de préférence une roue dentée, et l'appareil de rotation (40) présente une section dentée, de préférence une crémaillère, qui s'engrènent l'une dans l'autre pour la rotation du mécanisme de rotation ; ou
le mécanisme de rotation (38) présente un rouleau et l'appareil de rotation (40) présente une surface de roulement pour le rouleau ; ou
le mécanisme de rotation (38) interagit au moyen d'une force magnétique avec l'appareil de rotation (40) pour la rotation du mécanisme de rotation (38).

9. Dispositif (10A-10E) selon l'une des revendications précédentes, dans lequel :
le système d'entraînement plan (16) est réalisé pour faire tourner les dispositifs de déplacement (24) autour d'un axe normal distinct par rapport à l'élément formant base (22) pendant le déplacement des ébauches de récipients (12) à travers le four (18), de préférence pour le chauffage uniforme des ébauches de récipients (12).

10. Dispositif (10A-10E) selon l'une des revendications précédentes, dans lequel :
le système d'entraînement plan (16) est réalisé pour effectuer un déplacement de levage du dispositif de déplacement (24) par rapport à l'élément formant base (22) lors d'une prise en charge d'une ébauche de récipient (12) par un dispositif de déplacement (24) ou lors d'un transfert d'une ébauche de récipient (12) depuis un dispositif de déplacement (24), de sorte que, de préférence, un moyen de maintien (36) du dispositif de déplacement (24) s'enfonce dans l'ébauche de récipient (12) ou émerge de l'ébauche de récipient (12).

11. Dispositif (10A-10E) selon l'une des revendications précédentes, dans lequel :
le système d'entraînement plan (16) est réalisé pour déplacer un flux d'ébauches de récipients (12), transféré à des intervalles irréguliers aux dispositifs de déplacement (24), à des intervalles réguliers à travers le four (18).

12. Dispositif (10A-10E) selon l'une des revendications précédentes, dans lequel :
le système d'entraînement plan (16) est réalisé pour adapter une longueur d'une ligne de chauffage du déplacement des ébauches de récipients (12) à travers le four (18) au moyen des dispositifs de déplacement (24), de préférence en fonction d'un débit d'ébauches de récipients souhaité ; et/ou
le système d'entraînement plan (16) est réalisé pour adapter un temps de séjour des ébauches de récipients (12) dans le four (18) au moyen de l'adaptation d'une vitesse des dispositifs de déplacement (24), de préférence en fonction d'un débit d'ébauches de récipients souhaité.

13. Dispositif (10B ; 10C) selon l'une des revendications précédentes, dans lequel :
le four (18) présente plusieurs canaux de chauffage (26), de préférence pouvant être activés individuellement, à travers lesquels les ébauches de récipients (12) peuvent être déplacées de manière flexible au moyen des dispositifs de déplacement (24), de préférence en fonction d'une longueur respectivement souhaitée d'une ligne de chauffage du déplacement des ébauches de récipients (12) à travers le four (18).

14. Dispositif (10D) selon l'une des revendications précédentes, présentant en outre :
un appareil de stérilisation (30) pour la stérilisation des ébauches de récipients (12), de préférence au moyen de peroxyde d'hydrogène ou d'un rayonnement électromagnétique,
dans lequel les ébauches de récipients (12) peuvent être déplacées à travers l'appareil de stérilisation (30) au moyen des dispositifs de déplacement (24), de préférence en aval du four (18).

15. Dispositif (10A-10E) selon l'une des revendications précédentes, présentant en outre :
une machine de soufflage (20), de préférence une machine de soufflage bi-orienté, pour le soufflage de récipients à partir des ébauches de récipients (12), dans lequel la machine de soufflage (20) est disposée en aval du four (18) et/ou est réalisée pour la prise en charge d'ébauches de récipients (12) depuis les dispositifs de déplacement (24).
